(19) Europäisches Patentamt
European Patent Office
Office européen des brevets

(11) Numéro de publication : **0 003 199**
**B1**

(12) **FASCICULE DE BREVET EUROPÉEN**

(45) Date de publication du fascicule du brevet : **11.11.81**

(21) Numéro de dépôt : **79400019.0**

(22) Date de dépôt : **09.01.79**

(51) Int. Cl.$^3$ : **C 07 D401/04**, A 61 K 31/445 // (C07D401/04, 211/68)

(54) **Nouveaux dérivés du tétrahydro pyridinyl indole et leurs sels, le procédé de préparation, l'application à titre de médicaments de ces nouveaux produits et les compositions pharmaceutiques les renfermant.**

(30) Priorité : **16.01.78 FR 7801083**

(43) Date de publication de la demande : **25.07.79 (Bulletin 79/15)**

(45) Mention de la délivrance du brevet : **11.11.81 Bulletin 81/45**

(84) Etats contractants désignés : **BE CH DE GB IT LU NL SE**

(56) Documents cités :
**FR - A - 2 227 873**

**Journal of Organic Chemistry, 1975, vol. 40, n° 17, p. 2527, tabl. Ia, ligne 9**

(73) Titulaire : **ROUSSEL-UCLAF**
**102, route de Noisy Boîte postale no.9**
**F-93230 Romainville (FR)**

(72) Inventeur : **Nedelec, Lucien**
**45, boulevard de L'ouest**
**F-93340 Le Raincy (FR)**
Inventeur : **Guillaume, Jacques**
**11, Allée Hélène Boucher**
**F-93270 Sevran (FR)**
Inventeur : **Dumont, Claude**
**33, rue du Maréchal Vaillant**
**F-94130 Nogent sur Marne (FR)**

(74) Mandataire : **Vieillefosse, Jean-Claude et al**
**boîte postale no 9 102, route de Noisy**
**F-93230 Romainville (FR)**

**EP 0 003 199 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 17, rue du Louvre, 75001 Paris, France

Nouveaux dérivés du tétrahydro pyridinyl indole et leurs sels, le procédé de préparation, l'application à titre de médicaments de ces nouveaux produits et les compositions pharmaceutiques les renfermant.

La présente invention concerne de nouveaux dérivés du tétrahydropyridinyl indole et leurs sels, le procédé de préparation, l'application à titre de médicaments de ces nouveaux produits et les compositions pharmaceutiques les renfermant.

L'article du "Journal of Organic Chemistry", 1975, vol. 40, n° 17, pages 2 525 et suivantes, avait déjà décrit d'un point de vue chimique un certain nombre de dérivés du tétrahydropyridinyl-indole, sans indiquer d'utilisation pour ces produits.

Les brevets français n° 2 293 931 et 2 328 468 ont également décrit des dérivés du tétrahydropyridinyl-indole doués de propriétés psychostimulantes susceptibles d'applications dans le traitement des troubles dépressifs, et de propriétés antiparkinsonniennes ; ces produits sont dépourvus de propriétés neuroleptiques.

L'invention a pour objet de nouveaux dérivés du tétrahydropyridinyl indole et leurs sels d'addition avec les acides minéraux ou organiques, caractérisés en ce qu'ils répondent à la formule générale :

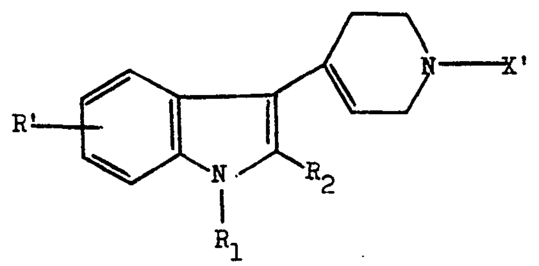

(I')

dans laquelle R' représente un atome d'hydrogène, un atome d'halogène ou un radical alcoxy renfermant de 1 à 3 atomes de carbone, $R_1$ et $R_2$, identiques ou différents, représentent un atome d'hydrogène ou un radical alcoyle renfermant de 1 à 3 atomes de carbone, X' représente un radical alcoyle renfermant de 1 à 6 atomes de carbone, un radical cycloalcoyle renfermant de 4 à 7 atomes de carbone, un radical alcényle ou alcynyle renfermant de 2 à 5 atomes de carbone, ou un radical aralcoyle renfermant de 7 à 12 atomes de carbone, étant entendu que si X' représente un radical méthyle, un au moins des substituants R', $R_1$ et $R_2$ comportent plus d'un atome de carbone et que si X' représente un radical benzyle, un au moins des substituants R', $R_1$ et $R_2$ ne représente pas un atome d'hydrogène et R' ne représente pas un atome d'hydrogène lorsque $R_1$ et $R_2$ représentent un radical méthyle.

L'invention a notamment pour objet les nouveaux dérivés du tétrahydropyridinyl-indole et leurs sels d'addition avec les acides minéraux ou organiques, caractérisés en ce qu'ils répondent à la formule générale :

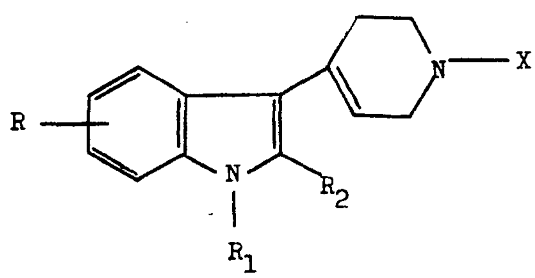

(I)

dans laquelle R représente un atome d'hydrogène ou un radical alcoxy renfermant de 1 à 3 atomes de carbone, $R_1$ et $R_2$, identiques ou différents, représentent un atome d'hydrogène ou un radical alcoyle renfermant de 1 à 3 atomes de carbne, X représente un radical alcoyle renfermant de 1 à 6 atomes de carbone, étant entendu que si X représente un radical méthyle, un au moins des substituants R, $R_1$, et $R_2$ a plus d'un atome de carbone.

Dans les formules (I) et (I') et dans ce qui suit, les substituants R et R' peuvent être en position 5 ou en position 6 de l'indole ; le radical alcoxy renfermant de 1 à 3 atomes de carbone désigne, par exemple, un radical méthoxy, éthoxy ou propoxy ; l'atome d'halogène peut être, par exemple, un atome de chlore, de brome ou de fluor ; le radical alcoyle renfermant de 1 à 3 atomes de carbone désigne, par exemple, un radical méthyle, éthyle ou propyle ; le radical cycloalcoyle renfermant de 4 à 7 atomes de carbone désigne, par exemple, un radical méthyl cyclopropyle ou un radical cyclopentyle ou cyclohexyle ; le radical alcényle renfermant de 2 à 5 atomes de carbone désigne, par exemple, un radical vinyle, allyle, butèn-2-yle ou pentèn-2-yle ; le radical alcynyle renfermant de 2 à 5 atomes de carbone désigne, par exemple, un radical propargyle ; le radical aralcoyle désigne, par exemple, un radical benzyle ou

phénéthyle ; le radical alcoyle renfermant de 1 à 6 atomes de carbone désigne, par exemple, un radical méthyle, éthyle, propyle, isopropyle, butyle, isobutyle, pentyle, isopentyle. Les sels d'addition avec les acides minéraux ou organiques peuvent être, par exemple, les sels formés avec les acides chlorhydrique, bromhydrique, iodhydrique, nitrique, sulfurique, phosphorique, acétique, formique, benzoïque, maléique, fumarique, succinique, tartrique, citrique, oxalique, glyoxylique, aspartique, alcanesulfoniques, tels que l'acide méthane sulfonique, et arylsulfoniques, tels que l'acide benzène sulfonique.

Parmi les nouveaux dérivés du tétrahydropyridinyl indole, objet de la présente invention, on peut citer, notamment, ceux répondant à la formule générale (I') ci-dessus, ainsi que leurs sels d'addition avec les acides minéraux ou organiques, lesdits dérivés étant caractérisés en ce que R' représente un atome d'halogène, $R_1$ et $R_2$ représentant un atome d'hydrogène ou un radical méthyle et X' représente un radical alcoyle renfermant de 2 à 6 atomes de carbone et, parmi ceux-ci, les dérivés caractérisés en ce que R' représente un atome de chlore en position 5, $R_1$ et $R_2$ représentent un atome d'hydrogène et X' représente un radical alcoyle renfermant de 2 à 6 atomes de carbone.

Parmi les nouveaux dérivés du tétrahydropyridinyl indole, objet de la présente invention, on peut également citer ceux répondant à la formule générale (I) ci-dessus, ainsi que leurs sels d'addition avec les acides minéraux ou organiques, lesdits dérivés étant caractérisés en ce que R représente un atome d'hydrogène ou un radical méthoxy, $R_1$ et $R_2$, identiques ou différents, représentent un atome d'hydrogène ou un radical méthyle et X représente un radical alcoyle renfermant de 2 à 6 atomes de carbone.

Parmi ces derniers dérivés, on retient plus particulièrement les dérivés du tétrahydropyridinyl indole répondant à la formule générale (I), ainsi que leurs sels d'addition avec les acides minéraux ou organiques, lesdits dérivés étant caractérisés en ce que R représente un atome d'hydrogène ou un radical méthoxy en position 5, $R_1$ et $R_2$ représentent un atome d'hydrogène et X représente un radical alcoyle renfermant de 2 à 6 atomes de carbone.

Parmi les produits préférés de l'invention, on retient, tout particulièrement, les produits préparés ci-après dans les exemples et notamment ceux dont les noms suivent :

— Le chlorhydrate du 3-(1-propyl 1,2,3,6-tétrahydro pyridin-4-yl) 1H-indole ;
— Le fumarate neutre du 5-méthoxy 3-(1-propyl 1,2,3,6-tétrahydropyridin-4-yl) 1H-indole ;
— Le chlorhydrate du 3-(1-pentyl 1,2,3,6-tétrahydro pyridin-4-yl) 1H-indole, ainsi que
— le chlorhydrate de 5-chloro 3-(1-propyl 1,2,3,6-tétrahydropyridin-4-yl) 1H-indole.

L'invention a également pour objet un procédé de préparation des dérivés tels que définis par la formule générale (I') ci-dessus, ainsi que de leurs sels, ledit procédé étant caractérisé en ce que l'on fait réagir un produit de formule :

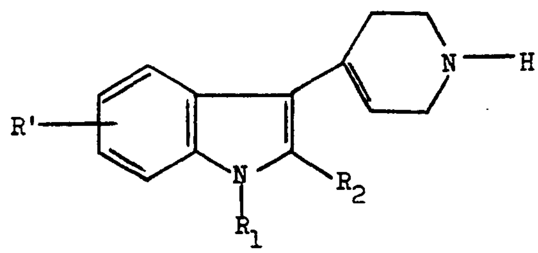

(II')

dans laquelle R', $R_1$, $R_2$ ont la signification déjà indiquée, avec un halogénure d'alcoyle de formule :

Hal—X' (III')

dans laquelle Hal représente un atome de chlore, de brome ou d'iode et X' a la signification déjà indiquée, pour obtenir un produit de formule (I') que l'on isole et, le cas échéant, traite par un acide pour en former le sel.

L'invention a notamment pour objet un procédé tel que défini ci-dessus, pour la préparation des produits de formule I, telle que définie précédemment, caractérisé en ce que l'on fait réagir un produit de formule

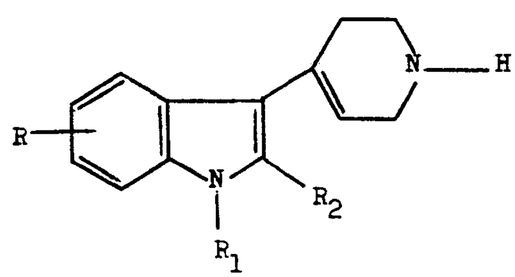

(II)

dans laquelle R, $R_1$ et $R_2$ ont la signification déjà indiquée, avec un halogénure d'alcoyle de formule

$$Hal—X \quad (III)$$

dans laquelle Hal et X ont la signification déjà indiquée, pour obtenir un produit de formule I que l'on isole et, le cas échéant, traite par un acide pour en former le sel.

Dans des conditions préférentielles de mise en œuvre de l'invention, le procédé de préparation ci-dessus décrit est caractérisé en ce que la réaction du produit de formule (II) ou (II') avec l'halogénure d'alcoyle de formule (III) ou (III') est effectuée au sein d'un solvant organique tel que l'acétone, en présence d'oxyde d'argent ou de carbonate de sodium.

La réaction peut également être effectuée au sein de la triéthylamine en présence d'hexamétapol.

Les dérivés de formule (I) ou (I') présentant un caractère basique. On peut avantageusement préparer les sels d'addition de ces dérivés en faisant réagir, en proportions sensiblement stœchiométriques, un acide minéral ou organique avec lesdits dérivés.

Les produits, objet de la présente demande, possèdent de très intéressantes propriétés pharmacologiques ; ils sont doués, notamment, de certaines propriétés à la fois antidépressives et neuroleptiques, ainsi que de propriétés antiémétiques.

Ces propriétés sont illustrées plus loin dans la partie expérimentale.

Ces propriétés justifient l'utilisation des nouveaux dérivés du tétrahydropyridinyl indole selon l'invention et de leurs sels, à titre de médicaments.

La présente demande a ainsi, également, pour objet l'application, à titre de médicaments, des nouveaux dérivés du tétrahydropyridinyl indole, tels que définis par la formule générale (I') et notamment (I), ainsi que de leurs sels d'addition avec les acides pharmaceutiquement acceptables.

Parmi les médicaments, selon l'invention, on retient, notamment, les médicaments caractérisés en ce qu'ils sont constitués par les nouveaux dérivés du tétrahydropyridinyl indole, répondant à la formule générale (I') ainsi que leurs sels d'addition avec les acides minéraux ou organiques pharmaceutiquement acceptables, formule générale (I') dans laquelle R' représente un atome d'halogène, $R_1$ et $R_2$ représentent un atome d'hydrogène ou un radical méthyle et X' représente un radical alcoyle renfermant de 2 à 6 atomes de carbone et notamment par les dérivés de formule générale (I') dans laquelle R' représente un atome de chlore en position 5, $R_1$ et $R_2$ représentent un atome d'hydrogène et X' représente un radical alcoyle renfermant de 2 à 6 atomes de carbone.

Parmi les médicaments selon l'invention, on retient également les médicaments caractérisés en ce qu'ils sont constitués par les nouveaux dérivés du tétrahydropyridinyl indole, répondant à la formule générale (I), ainsi que leurs sels d'addition avec les acides minéraux ou organiques pharmaceutiquement acceptables, formule générale (I) dans laquelle R représente un atome d'hydrogène ou un radical méthoxy, $R_1$ et $R_2$, identiques ou différents, représentent un atome d'hydrogène ou un radical méthyle et X représente un radical alcoyle renfermant de 2 à 6 atomes de carbone.

Parmi ces derniers, on retient tout particulièrement ceux répondant à la formule générale (I) dans laquelle R représente un atome d'hydrogène ou un radical méthoxy en position 5, $R_1$ et $R_2$ représentent un atome d'hydrogène et X représente un radical alcoyle renfermant de 2 à 6 atomes de carbone.

Parmi les médicaments préférés de l'invention, on retient, tout particulièrement :

— Le chlorhydrate du 3-(1-propyl 1,2,3,6-tétrahydro pyridin-4-yl) 1H-indole ;
— Le fumarate neutre du 5-méthoxy 3-(1-propyl 1,2,3,6-tétrahydropyridin-4-yl) 1H-indole ;
— Le chlorhydrate du 3-(1-pentyl 1,2,3,6-tétrahydro pyridin-4-yl) 1H-indole ;
— Le chlorhydrate de 5-chloro 3-(1-propyl 1,2,3,6-tétrahydropyridin-4-yl) 1H-indole.

Ces médicaments trouvent par exemple leur emploi dans le traitement des troubles psychiques, des troubles du comportement, des troubles caractériels ainsi que dans le traitement des vomissements et nausées de toutes origines.

La dose usuelle, variable selon le produit utilisé, le sujet traité et l'affection en cause, peut être, par exemple, de 5 mg à 200 mg par jour, par voie orale chez l'homme.

L'invention a enfin pour objet les compositions pharmaceutiques qui renferment, au moins, un dérivé précité ou l'un de ses sels d'addition avec les acides pharmaceutiquement acceptables, à titre de principe actif.

A titre de médicaments, les dérivés du tétrahydropyridinyl indole selon l'invention et leurs sels d'addition avec les acides pharmaceutiquement acceptables, peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les capsules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (II) ou (II'), lorsqu'ils ne sont pas connus, peuvent être préparés par réaction

d'un produit de formule :

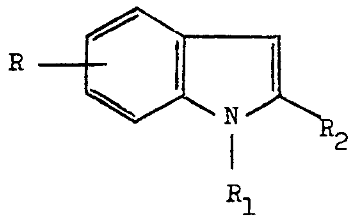

(IV)

ou

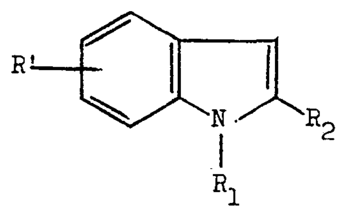

(IV')

dans laquelle R, R', $R_1$ et $R_2$ ont la signification déjà indiquée, avec du chlorhydrate de 4-pipéridone, dans l'acide acétique, en présence ou non d'un acide fort ou en milieu basique, notamment dans la potasse méthanolique. On opère de préférence en milieu acide quand $R_1$ représente un radical alcoyle.

Des exemples de telles préparations figurent ci-après dans la partie expérimentale.

La préparation de certains de ces dérivés figure dans la demande française publiée n° 2 362 628 ou dans une demande de premier certificat d'addition à ladite demande, déposée ce jour par la demanderesse et intitulée « Nouveaux dérivés du pipéridyl indole et leurs sels, procédé de préparation et application à titre de médicaments. »

Il va être donné maintenant, à titre non limitatif, des exemples de mise en œuvre de l'invention.

Exemple 1

Fumarate neutre du 5-méthoxy 3-(1-propyl) 1,2,3,6-tétrahydropyridin-4-yl 1H-indole

Stade a :

5-méthoxy 3-(1-propyl 1,2,3,6-tétrahydropyridin-4-yl) 1H-indole.

On met en suspension 6 g de 5-méthoxy 3-(1,2,3,6 tétrahydropyridin-4-yl) 1H-indole, dans une solution renfermant 60 $cm^3$ de triéthylamine, 6 $cm^3$ d'hexaméthylphosphotriamide et 3 $cm^3$ d'iodure de propyle. On porte la suspension obtenue à 80 °C. pendant une demi-heure, rajoute 0,5 $cm^3$ d'iodure de propyle et maintient à 80 °C pendant 30 minutes. On refroidit et ajoute 10 $cm^3$ d'eau et 20 $cm^3$ de méthanol. On élue au chlorure de méthylène et décante. On extrait les fractions aminées à l'acide chlorhydrique N et alcalinise la phase acide par addition de carbonate de sodium. On extrait au chlorure de méthylène, lave la phase organique et sèche. On obtient ainsi 6 g de produit que l'on dissout dans 5 $cm^3$ de méthanol, on ajoute 15 $cm^3$ d'acétate d'éthyle, on filtre les cristaux formés, les rince à l'acétate d'éthyle et les sèche. On obtient ainsi le produit recherché fondant à 164 °C.

Stade B :

Fumarate neutre du 5-méthoxy 3-(1-propyl 1,2,3,6-tétrahydropyridin-4-yl) 1H-indole.

On dissout 3,1 g du produit préparé au stade A dans 15 $cm^3$ de méthanol. On verse la solution obtenue dans une solution renfermant 15 $cm^3$ de méthanol et 0,68 g d'acide fumarique. On évapore le méthanol sous pression réduite et ajoute 20 $cm^3$ d'isopropanol. On maintient sous agitation pendant 30 minutes à la température ambiante et pendant 30 minutes à 0 °C. On filtre, rince à l'isopropanol et sèche. On obtient 3,5 g de produit brut que l'on purifie par cristallisation. On obtient ainsi 2,13 g de produit recherché fondant à 200 °C.

ANALYSE : $C_{38}H_{48}N_4O_6$ = 656,836

| | | | | | | |
|---|---|---|---|---|---|---|
| Calculé : | C % | 69,49 | H % | 7,37 | N % | 8,53 |
| Trouvé : | | 69,7 | | 7,7 | | 8,6 |

Le 5-méthoxy 3-(1,2,3,6-tétrahydropyridin-4-yl) 1H-indole, utilisé comme produit de départ peut être préparé comme suit :

On dissout, à 100 °C, 12,6 g de 5-méthoxy indole dans 240 $cm^3$ d'acide acétique, ajoute 44 g de

chlorhydrate de 4-pipéridone monohydratée et maintient le chauffage 30 minutes à 100 °C.

Après refroidissement, on verse sur de l'eau glacée additionnée de 400 $cm^3$ d'ammoniaque concentrée, extrait à l'acétate d'éthyle et lave la phase organique à l'eau salée. On sèche sur sulfate de magnésium et évapore à sec. On obtient 20 g de produit brut que l'on purifie par chromatographie sur silice en éluant au mélange chloroforme-méthanol-triéthylamine (7-2-1).

On obtient 5,26 g de 5-méthoxy 3-(1,2,3,6-tétrahydropyridin-4-yl) 1H-indole sous forme d'une résine.

Exemple 2

Chlorhydrate du 3-(1-propyl 1,2,3,6-tétrahydropyridin-4-yl) 1H indole

Stade A :

3-(1-propyl 1,2,3,6-tétrahydropyridin-4-yl) 1H indole. On dissout 4,5 g de 3-(1,2,3,6-tétrahydropyridin-4-yl) 1H-indole dans 90 $cm^3$ d'acétone. On ajoute à la solution obtenue 3,15 g d'oxyde d'argent et 2,25 $cm^3$ d'iodure de propyl. On chauffe pendant 3 heures à 50 °C, refroidit, filtre et distille à sec sous vide. On chromatographie sur silice le produit brut obtenu en éluant par le mélange chloroforme, acétone, triéthylamine (6-3-1). On distille les fractions et redissout le résidu dans 200 $cm^3$ d'acétate d'éthyle au reflux ; on filtre et concentre à 150 $cm^3$. On amène à 20 °C, amorce la cristallisation et glace une nuit. On essore, lave à l'acétate d'éthyle et sèche. On obtient ainsi 3,9 g du produit recherché fondant à 210 °C.

Stade B :

Chlorhydrate du 3-(1-propyl 1,2,3,6-tétrahydropyridin-4-yl) 1H indole.

On dissout les 3,9 g de produit obtenu au stade A dans 250 $m^3$ d'isopropanol au reflux et ajoute, goutte à goutte, une solution d'acide chlorhydrique gazeux dans l'isopropanol jusqu'à l'obtention d'un pH acide. On glace pendant deux heures, essore, lave à l'isopropanol et sèche sous pression réduite. On obtient 4,15 g de produit brut que l'on purifie par cristallisation. On obtient ainsi 3,55 g de produit recherché fondant à 230 ~ 232 °C.

ANALYSE : $C_{16}H_{20}N_2HCl$ = 276,821

| | | | | | | | | |
|---|---|---|---|---|---|---|---|---|
| Calculé : | C % | 69,42 | H % | 7,65 | Cl % | 12,81 | N % | 10,12 |
| Trouvé : | | 69,5 | | 7,7 | | 12,8 | | 9,9 |

Le 3-(1,2,3,6-tétrahydropyridin-4-yl) 1H-indole utilisé comme produit de départ peut être préparé comme suit :

On dissout 10 g d'indole dans 200 $cm^3$ d'acide acétique, chauffe à 95-100 °C sous agitation et atmosphère d'azote. On ajoute 50 $cm^3$ d'acide phosphorique aqueux N et 39,3 g de chlorhydrate de 4-pipéridone monohydratée. On chauffe à 100 °C pendant une heure, laisse refroidir, verse sur de la glace additionnée de 350 $cm^3$ d'ammoniaque concentrée et extrait à l'acétate d'éthyle. On lave à l'eau, à l'eau salée, sèche sur sulfate de magnésium et évapore à sec. On obtient 14,7 g de produit brut que l'on empâte sous azote avec 75 $cm^3$ de méthanol. On filtre sous vide, rince au méthanol et à l'éther. On obtient 1,42 g de 3-(1,2,3,6-tétrahydropyridin-4-yl) 1H-indole. (F = 185-186 °C).

On évapore les liqueurs mères et purifie par chromatographie sur silice le produit brut obtenu en éluant au mélange chloroforme-méthanol-triéthylamine (6-3-1). On recueille 4,55 g de produit de Rf égal à 0,15 que l'on empâte dans l'éther. On obtient finalement 4,295 g de 3-(1,2,3,6-tétrahydro pyridin-4-yl) 1H-indole, soit au total avec le premier jet 5,715 g. On purifie le produit obtenu par chaud et froid dans l'isopropanol et obtient 3,56 g de 3-(1,2,3,6-tétrahydro pyridin-4-yl) 1H-indole (F = 190-191 °C).

Exemple 3

Chlorhydrate du 3-(1-pentyl 1,2,3,6-tétrahydropyridin-4-yl) 1H-indole

Stade A :

3-(1-pentyl 1,2,3,6-tétrahydropyridin-4-yl) 1H-indole.

On introduit 10 g de 3-(1,2,3,6-tétrahydropyridin-4-yl) 1H-indole dans 100 $cm^3$ d'acétone, ajoute 5,18 g d'oxyde d'argent puis 20 $cm^3$ d'iodure de pentyle.

Après 4 heures d'agitation à température ambiante, on ajoute à nouveau 2,5 $cm^3$ d'iodure de pentyle et poursuit l'agitation pendant 1 heure.

On filtre, lave par le mélange chlorure de méthylène-méthanol (50/50) et élimine les solvants sous pression réduite à 40 °C. On purifie le produit brut par chromatographie sur gel de silice en éluant par le mélange chloroforme-méthanol-triéthylamine (7-2-1). Après avoir chassé l'éluant on redissout l'extrait sec obtenu dans 250 $cm^3$ d'éthanol au reflux, filtre et laisse cristalliser à 0-5 °C. On obtient finalement 7,9 g du produit recherché, fondant à environ 180 °C.

Stade B :

Chlorhydrate du 3-(1-pentyl 1,2,3,6-tétrahydropyridin-4-yl) 1H-indole.

On met en suspension 7,9 g de produit obtenu au stade A, dans 100 $cm^3$ d'éthanol absolu puis introduit à 0-5 °C, 30 $cm^3$ d'une solution saturée d'acide chlorhydrique dans l'éthanol. On laisse 1 heure sous agitation, filtre, lave à l'éther puis sèche sous vide à 40-50 °C. On obtient 8,45 g du chlorhydrate attendu, sous forme d'un produit jaune fondant à 210° puis 240 °C.

ANALYSE : $C_{18}H_{25}N_2Cl$ = 304,866

| | | | | | | | | |
|---|---|---|---|---|---|---|---|---|
| Calculé : | C % | 70,9 | H % | 8,3 | N % | 9,2 | Cl % | 11,6 |
| Trouvé : | | 70,7 | | 8,3 | | 9,2 | | 11,6 |

Exemple 4

Chlorhydrate de 3-(1-éthyl 1,2,3,6-tétrahydro pyridin-4-yl) 1H-indole

Stade A :

3-(1-éthyl 1,2,3,6-tétrahydro pyridin-4-yl) 1H-indole

On met en contact à 30-35 °C pendant 5 heures, sous atmosphère inerte, 9,91 g de 3-(1,2,3,6-tétrahydro pyridin-4-yl) 1H-indole avec 10,6 g de carbonate de sodium 150 $cm^3$ de diméthylformamide et 4,5 $cm^3$ de bromure d'éthyle, puis verse dans 1,5 litre d'eau et le produit précipite. On agite 1 heure, filtre, rince à l'eau le précipité et sèche une nuit en étuve à 70 °C sous vide en présence d'un déshydratant.

On obtient ainsi 8,95 g du produit recherché, brut, fondant à 205 °C, que l'on redissout dans 400 $cm^3$ d'acétate d'éthyle au reflux, filtre à chaud, concentre à 250 $cm^3$, cristallise, laisse une heure, filtre, rince à l'acétate d'éthyle et obtient 6,28 g de cristaux jaunes fondant à 205 °C.

On concentre au tiers les liqueurs mères et obtient encore 1,76 g du produit cherché par cristallisation, soit un total de 8,04 g.

Stade B :

Chlorhydrate du 3-(1-éthyl 1,2,3,6-tétrahydropyridin-4-yl) 1H-indole

On met en suspension les 8,04 g du produit obtenu au Stade A dans 80 $cm^3$ d'éthanol, glace, additionne jusqu'à pH acide de l'acide chlorhydrique en solution dans l'éthanol et laisse précipiter. Après 1 h 30, on filtre, rince à l'éthanol, sèche à température ambiante sous vide, obtient 8,67 g du chlorhydrate brut cherché que l'on purifie par recristallisation à deux reprises dans l'éthanol et obtient 6,21 g du produit cherché pur, fondant à 232-233 °C.

ANALYSE : $C_{15}H_{19}ClN_2$ = 262,789

| | | | | | | | | |
|---|---|---|---|---|---|---|---|---|
| Calculé : | C % | 69,42 | H % | 7,65 | Cl % | 12,81 | N % | 10,12 |
| Trouvé : | | 69,5 | | 7,7 | | 12,8 | | 9,9 |

Exemple 5

Chlorhydrate de 3-(1-méthyl éthyl 1,2,3,6-tétrahydro pyridin-4-yl) 1H-indole

Stade A :

3-(1-méthyl éthyl 1,2,3,6-tétrahydropyridin-4-yl) 1H-indole

On agite sous atmosphère inerte 5 g de 3-(1,2,3,6-tétrahydro pyridin-4-yl) 1H-indole, 50 $cm^3$ de diméthylformamide, 5,25 g de carbonate de sodium et 2,7 $cm^3$ de 2-iodopropane ajouté goutte à goutte, après 20 heures dans 400 $cm^3$ d'eau, agite 30 minutes, cristallise, essore, lave, sèche à 50 °C sous vide et récupère 5,8 g du produit cherché fondant à 178 °C.

Stade B :

Chlorhydrate de 3-(1-méthyl éthyl 1,2,3,6-tétrahydro pyridin-4-yl) 1H-indole.

On dissout dans 150 $cm^3$ d'isopropanol à chaud le produit obtenu au Stade A, ajoute goutte à goutte une solution d'acide chlorhydrique gazeux dans l'isopropanol jusqu'à pH acide, cristallise, glace une nuit, essore, lave à l'isopropanol, sèche sous vide et obtient 6 g du produit cherché fondant à 260 °C.

ANALYSE : (après recristallisation dans l'éthanol). $C_{16}H_{21}ClN_2$ = 276,821

| | | | | | | | | |
|---|---|---|---|---|---|---|---|---|
| Calculé : | C % | 69,42 | H % | 7,65 | Cl % | 12,81 | N % | 10,12 |
| Trouvé : | | 69,2 | | 7,9 | | 12,7 | | 10,1 |

Exemple 6

Chlorhydrate du 6-méthoxy 3-(1-propyl 1,2,3,6-tétrahydro pyridin-4-yl) 1H-indole

Stade A :

6-méthoxy 3-(1-propyl 1,2,3,6-tétrahydro pyridin-4-yl) 1H-indole

On agite sous atmosphère inerte 10 g de 6-méthoxy 3-(1,2,3,6-tétrahydro pyridin-4-yl) 1H-indole, 9,28 g de carbonate de sodium et 8,93 g d'iodure de propyle dans 200 $cm^3$ de diméthylformamide, après 5 heures verse dans 1,5 l d'eau, cristallise, agite 1 heure, filtre sous vide, rince à l'eau, sèche sous vide à 50 °C en présence de déshydratant et obtient 9,9 g du produit cherché fondant à 219-220 °C.

Stade B :

Chlorhydrate du 6-méthoxy 3-(1-propyl 1,2,3,6-tétrahydro pyridin-4-yl) 1H indole

On met en suspension 7,3 g du produit obtenu au stade précédent et après traitement au charbon actif dans 105 $cm^3$ d''éthanol, refroidit dans la glace, ajoute 7,5 $cm^3$ d'une solution saturée d'acide chlorhydrique gazeux dans l'éthanol jusqu'à pH acide, agite 1 h 30 à 0-5 °C, essore, rince à l'éthanol,

sèche sous vide à 50 °C, obtient 8,25 g de produit brut qui, recristallisé dans l'eau, donne 6,2 g du produit cherché pur, fondant à plus de 260 °C.

ANALYSE : $C_{17}H_{23}ClN_2O$ = 306,843

| | | | | | | | | |
|---|---|---|---|---|---|---|---|---|
| Calculé : | C % | 66,54 | H % | 7,55 | Cl % | 11,55 | N % | 9,12 |
| Trouvé : | | 66,2 | | 7,7 | | 11,7 | | 9,0 |

Le 6-méthoxy 3-(1,2,3,6-tétrahydropyridin-4-yl) 1H-indole de départ peut être préparé de la façon suivante :

On porte au reflux sous azote pendant 8 h 30, 20 g de 6-méthoxy 1H-indole avec 41,75 g d'hydrate de chlorhydrate de 4-pipéridone dans 205 $cm^3$ de potasse méthanolique 2N, puis agite une nuit à température ambiante, dilue lentement à l'eau jusqu'à 1,2 litre, cristallise, agite 30 minutes, filtre, rince soigneusement à l'eau, sèche et obtient 23,05 g du produit cherché fondant à 193-194 °C.

Exemple 7

Chlorhydrate de 3-/1-(2-phényléthyl) 1,2,3,6-tétrahydro pyridin-4-yl/1H-indole

Stade A :

3-/1-(2-phényléthyl) 1,2,3,6-tétrahydro pyridin-4-yl/1H-indole

On agite pendant 5 heures à 45 °C, 6,94 g de 3-(1,2,3,6-tétrahydro pyridin-4-yl) 1H-indole dissous dans 105 $cm^3$ de diméthylformamide avec 7,42 g de carbonate de sodium et 5,95 $cm^3$ de bromure de phényléthyle, verse ensuite sous agitation dans 1 litre d'eau glacée, cristallise, agite 2 heures, filtre, rince à l'eau, laisse sécher une nuit sous vide en présence d'un déshydratant, obtient 10,23 g de produit jaune fondant à 203 °C que l'on purifie par cristallisation dans l'éthanol et récupère 7,33 g du produit cherché fondant à 206-207 °C.

Stade B :

Chlorhydrate de 3-/1-(2-phényléthyl) (1,2,3,6-tétrahydro pyridin-4-yl) 1H-indole

On met en suspension 8,24 g de produit tel qu'obtenu au Stade A dans 85 $cm^3$ d'éthanol glacé, ajoute sous agitation de l'acide chlorhydrique en solution dans l'éthanol jusqu'à pH acide, agite une heure, filtre, rince à l'éthanol, recristallise dans le méthanol et obtient en deux fois 6,99 g du produit pur cherché fondant à 280 °C.

ANALYSE : $C_{21}H_{23}ClN_2$ = 338,888

| | | | | | | | | |
|---|---|---|---|---|---|---|---|---|
| Calculé : | C % | 74,42 | H % | 6,84 | Cl % | 8,26 | N % | 10,46 |
| Trouvé : | | 74,4 | | 6,9 | | 7,9 | | 10,7 |

Exemple 8

Chlorhydrate de 3-/1-(2-propényl) 1,2,3,6-tétrahydro pyridin-4-yl/1H-indole

Stade A :

3-/1-(2-propényl) 1,2,3,6-tétrahydro pyridin-4-yl/1H-indole.

On agite pendant 1 heure à 32 °C une solution de 13,86 g de 3-(1,2,3,6-tétrahydro pyridin-4-yl) 1H-indole dans 210 $cm^3$ de diméthylformamide avec 14,84 g de carbonate de sodium et 7,3 $cm^3$ de bromure d'allyle redistillé sous atmosphère inerte, verse ensuite dans 2 litres d'eau, précipite, filtre après une nouvelle heure d'agitation, rince, sèche sous vide en présence de déshydratant, récupère 16,61 g de produit jaune pâle fondant à 177-179 °C que l'on purifie par recristallisation dans l'acétate d'éthyle, et obtient en trois fois 9,72 g du produit cherché pur fondant à 177-179 °C.

Stade B :

Chlorhydrate de 3-/1-(2-propényl) 1,2,3,6-tétrahydro pyridin-4-yl 1H-indole

On met en suspension 8,75 g de produit obtenu au Stade A dans 85 $cm^3$ d'éthanol glacé, ajoute jusqu'à pH acide une solution saturée d'acide chlorhydrique dans l'éthanol, agite 1 heure à 5 °C, filtre, rince à l'éthanol, sèche sous vide à 50 °C, obtient 9,36 g de produit qui, recristallisé dans l'éthanol, permet de récupérer 7,45 g du chlorhydrate cherché pur, fondant à 177-178 °C et à 217-218 °C.

ANALYSE : $C_{16}H_{19}N_2Cl$ = 274,8

| | | | | | | | | |
|---|---|---|---|---|---|---|---|---|
| Calculé : | C % | 69,93 | H % | 6,96 | N % | 10,19 | Cl % | 12,90 |
| Trouvé : | | 69,7 | | 7,1 | | 9,9 | | 13,0 |

Exemple 9

Chlorhydrate de 5-chloro 3-(1-propyl 1,2,3,6-tétrahydro pyridin-4-yl)- 1H-indole

Stade A :

5-chloro 3-(1-propyl 1,2,3,6-tétrahydro pyridin-4-yl) 1H-indole

On agite pendant 5 heures à température ambiante, sous atmosphère inerte, 9,28 g de 5-chloro 3-(1,2,3,6-tétrahydro pyridin-4-yl) 1H-indole dissous dans 140 $cm^3$ de diméthylformamide, avec 8,48 g de carbonate de sodium et 4,67 $cm^3$ d'iodure de propyle, verse ensuite sous agitation dans 1,4 l d'eau,

cristallise, filtre, rince à l'eau, sèche sous vide en présence d'un déshydratant, purifie par recristallisation dans l'éthanol et obtient 6,95 g du produit cherché fondant à 229-230 °C.

Stade B :

Chlorhydrate du 5-chloro 3-(1-propyl 1,2,3,6-tétrahydro pyridin-4-yl) 1H-indole

On met en suspension les 6,95 g du produit obtenu au Stade A dans 105 $cm^3$ d'éthanol, glace, ajoute jusqu'à pH acide de l'éthanol chlorhydrique en solution saturée, agite 2 h 30 à 0 °C, filtre, rince à l'éthanol, sèche en étuve, récupère 7,566 g de produit brut que l'on recristallise dans l'éthanol et obtient 5,047 g de cristaux jaune clair du produit cherché, fondant à 260 °C.

ANALYSE : $C_{16}H_{20}Cl_2N_2$ = 311,256

| | | | | | | | | |
|---|---|---|---|---|---|---|---|---|
| Calculé : | C % | 61,74 | H % | 6,47 | Cl % | 22,78 | N % | 8,99 |
| Trouvé : | | 61,6 | | 6,5 | | 22,7 | | 8,8 |

Le 5-chloro 3-(1,2,3,6-tétrahydro pyridin-4-yl) 1H-indole de départ peut être préparé selon un procédé décrit dans le brevet belge n° 858 101.

Exemple 10

Chlorhydrate du 5-chloro 3-(1-éthyl 1,2,3,6-tétrahydro pyridin-4-yl) 1H-indole

Stade A :

5-chloro 3-(1-éthyl 1,2,3,6-tétrahydro pyridin-4-yl) 1H-indole

On agite pendant 6 h 30 à température ambiante, sous atmosphère inerte, 12 g de 5-chloro 3-(1,2,3,6-tétrahydro pyridin-4-yl) 1H-indole dans 120 $cm^3$ de diméthylformamide anhydre avec 10,92 g de carbonate de sodium et 5,1 $cm^3$ de bromoéthane, puis lentement 350 $cm^3$ d'eau distillée, laisse précipiter, agite 1 heure 30, laisse au repos une nuit, filtre sous vide, empâte trois fois à l'eau et une fois avec 25 $cm^3$ d'éthanol à 50 % d'eau, sèche en présence de déshydratant, obtient 9,48 g du produit cherché jaune fondant à 208-210 °C, que l'on recristallise deux fois dans l'éthanol et récupère 7,487 g de produit cherché.

Stade B :

Chlorhydrate de 5-chloro 3-(1-éthyl 1,2,3,6-tétrahydro pyridin-4-yl) 1H-indole

On met en suspension les 7,48 g du produit obtenu au Stade A dans 40 $cm^3$ d'éthanol, refroidit à 0 °C, ajoute jusqu'à pH = 1 de l'éthanol chlorhydrique. Le produit précipité, on agite 30 mn à 0 °C, laisse à cette température 1 heure, filtre, rince à l'éthanol glacé, sèche sous vide à 50 °C, récupère 7,3 g du produit cherché fondant à 225 °C, que l'on recristallise dans l'éthanol, et obtient 5,9 g du produit pur cherché fondant à 225 °C.

ANALYSE : $C_{15}H_{18}Cl_2N_2$ = 297,239

| | | | | | | | | |
|---|---|---|---|---|---|---|---|---|
| Calculé : | C % | 60,61 | H % | 6,1 | Cl % | 23,85 | N % | 9,42 |
| Trouvé : | | 60,4 | | 6,1 | | 23,6 | | 9,3 |

Exemple 11

Chlorhydrate de 5-chloro 3-/1-(cyclopropyl méthyl) 1,2,3,6-tétrahydro pyridin-4-yl/1H-indole

Stade A :

5-chloro 3-/1-(cyclopropyl méthyl) 1,2,3,6-tétrahydro pyridin-4-yl/1H-indole.

On ajoute à une solution de 12,5 g de 5-chloro 3-(1,2,3,6-tétrahydropyridin-4-yl) 1H-indole dans 120 $cm^3$ de diméthylformamide anhydre, 11,5 g de carbonate de sodium et 6,5 $cm^3$ de chlorométhyl cyclopropane, agite 24 heures à 70 °C sous atmosphère inerte, laisse refroidir à 30 °C, ajoute 300 $cm^3$ d'eau distillée sous agitation en refroidissant à l'aide d'un bain d'eau glacée, laisse précipiter, agite 45 minutes, abandonne 15 minutes, essore, lave trois fois à l'eau distillée, empâte avec 20 $cm^3$ d'éthanol à 50 % d'eau, sèche sous vide à 50 °C en présence de déshydratant, récupère 13,54 g de produit brut que l'on redissout au reflux dans 350 $cm^3$ d'éthanol, filtre, concentre à 300 $cm^3$, cristallise, laisse revenir à température ambiante, laisse 1 h 30 au réfrigérateur, essore, rince à l'éthanol, sèche sous vide et obtient 7,8 g de produit cherché fondant à 205 °C.

Stade B :

Chlorhydrate de 5-chloro 3-/1-(cyclopropyl méthyl) 1,2,3,6-tétrahydro pyridin-4-yl/1H-indole

On ajoute à la suspension des 7,8 g du produit obtenu au stade précédent dans 80 $cm^3$ d'éthanol refroidi par un bain d'eau glacée, de l'éthanol chlorhydrique jusqu'à pH = 1, agite pendant 1 heure le précipité crème obtenu, laisse reposer 15 minutes, essore, rince à l'éthanol, sèche sous pression réduite, recristallise dans l'éthanol et obtient 6,9 g du chlorhydrate cherché pur, fondant à 242-244 °C.

ANALYSE : $C_{17}H_{20}Cl_2N_2$ = 323,268

| | | | | | | | | |
|---|---|---|---|---|---|---|---|---|
| Calculé : | C % | 63,16 | H % | 6,23 | Cl % | 21,93 | N % | 8,66 |
| Trouvé : | | 63,3 | | 6,2 | | 22,2 | | 8,6 |

Exemple 12

Chlorhydrate du 6-méthoxy 2-méthyl 3-(1-propyl 1,2,3,6-tétrahydro pyridin-4-yl) 1H-indole

Stade A :

6-méthoxy 2-méthyl 3-(1-propyl 1,2,3,6-tétrahydro pyridin-4-yl) 1H-indole

On agite à 40 °C sous atmosphère d'azote 10,3 g de chlorhydrate de 6-méthoxy 2-méthyl 3-(1,2,3,6-tétrahydro pyridin-4-yl) 1H-indole avec 11,845 g de carbonate de sodium, 103 cm³ de diméthylformamide et 4,82 cm³ d'iodure de propyle, après 4 h 30 verse sous agitation le mélange dans de l'eau, extrait à l'acétate d'éthyle la gomme obtenue, lave à l'eau, puis à l'eau salée, sèche, concentre à sec, empâte les 10,1 g de résine obtenue dans 50 cm³ d'éthanol, cristallise ainsi le produit, agite 45 minutes à température ambiante, glace 30 minutes sous agitation, maintient 30 minutes au repos, essore, rince à l'éthanol et à l'éther et obtient 7,8 g de la base du produit cherché fondant à 90-95 °C.

Stade B :

Chlorhydrate du 6-méthoxy 2-méthyl 3-(1-propyl 1,2,3,6-tétrahydro pyridin-4-yl) 1H-indole

On dissout 9,1 g de base telle qu'obtenue au stade précédent dans 70 cm³ d'isopropanol, filtre, glace la solution, ajoute de l'isopropanol saturé d'acide chlorhydrique sec, laisse précipiter, agite 30 minutes à 0 °C, laisse reposer 30 minutes, essore à température ambiante, rince à l'isopropanol, récupère 8,73 g du chlorhydrate cherché fondant au-delà de 260 °C purifie par recristallisation dans le méthanol et obtient 4,4 g du produit pur cherché, fondant à 275 °C.

ANALYSE : $C_{18}H_{25}ClN_2O$ = 320,87

| | | | | | | | | |
|---|---|---|---|---|---|---|---|---|
| Calculé : | C % | 67,37 | H % | 7,85 | Cl % | 11,05 | H % | 8,73 |
| Trouvé : | | 67,2 | | 8,00 | | 11, | | 8,6 |

Le chlorhydrate de 6-méthoxy 2-méthyl 3-(1,2,3,6-tétrahydro pyridin-4-yl) 1H-indole peut être préparé de la façon suivante :

On agite à 100 °C sous atmosphère inerte, 2 g de 6-méthoxy 2-méthyl 1H-indole avec 4 g d'hydrate de chlorhydrate de 4-pipéridone dans 40 cm³ d'acide acétique. Après 1 h 30, on refroidit, verse sur une solution de 150 g de glace et de 80 cm³ d'ammoniaque pure 22° Bé, extrait à l'acétate d'éthyle, lave à l'eau, sèche sur sultate de magnésium, distille à sec sous pression réduite, reprend le résidu à l'acétate d'éthyle, empâte à chaud, laisse refroidir, essore, lave à l'acétate d'éthyle, sèche sous pression réduite et obtient 2,5 g du produit brut.

Préparation du chlorhydrate

On dissout le produit obtenu dans 50 cm³ d'isopropanol à chaud, refroidit, ajoute goutte à goutte une solution d'acide chlorhydrique gazeux dans l'isopropanol jusqu'à pH acide, amorce la cristallisation. Après une nuit, on essore, lave à l'isopropanol, sèche sous vide et obtient 2,25 g du produit attendu, fondant à 270 °C.

ANALYSE : $C_{15}H_{19}ClN_2O$

| | | | | | | | | |
|---|---|---|---|---|---|---|---|---|
| Calculé : | C % | 64,52 | H % | 6,87 | Cl % | 12,72 | N % | 10,05 |
| Trouvé : | | 64,5 | | 7,0 | | 13,0 | | 9,9 |

## Exemple 13

Compositions pharmaceutiques

On a préparé des comprimés répondant à la formule :

— Chlorhydrate du 3-(1-propyl 1,2,3,6-tétrahydropyridin-4-yl) 1H-indole ........ 10 mg
— Excipient q.s. pour un comprimé terminé à ........ 200 mg
(détail de l'excipient : lactose, amidon, talc, stéarate de magnésium).

## Exemple 14

Compositions pharmaceutiques

On a préparé des comprimés répondant à la formule :

— Chlorhydrate de 3-(1-éthyl 1,2,3,6-tétrahydro pyridin-4-yl) 1H-indole ........ 25 mg
— Excipient q.s. pour un comprimé terminé à ........ 200 mg
(détail de l'excipient : lactose, amidon, talc, stéarate de magnésium).

## Exemple 15

Compositions pharmaceutiques

On a préparé des comprimés répondant à la formule :

— Chlorhydrate de 5-chloro 3-(1-propyl 1,2,3,6-tétrahydro pyridin-4-yl) 1H-indole ........ 25 mg
— Excipient q.s. pour un comprimé terminé à ........ 200 mg.
(détail de l'excipient : lactose, amidon, talc, stéarate de magnésium.

## ETUDE PHARMACOLOGIQUE

1) Potentialisation des stéréotypies à l'amphétamine :

Les essais sont réalisés sur des lots de 5 rats mâles de 150 à 180 g. Chaque animal est placé individuellement dans une cage grillagée (29 × 25 × 17 cm) contenant quelques débris de frisure de bois.

Un délai d'une heure est observé entre l'administration du composé étudié et l'injection, par voie intrapéritonéale, de 5 mg/kg de sulfate de dexamphétamine. Le comportement des animaux est noté ensuite toutes les 1/2 heures pendant 5 heures avec la cotation préconisée par HALLIWELL et Coll. (Brit. J. Pharmacol. 1964, *23,* 330-350). L'animal est endormi (0), il est éveillé mais immobile (1), il tourne dans la cage (2), il en renifle le couvercle (3), il en lèche les parois (4), il touche les copeaux ou les barreaux de la cage avec les dents (5), il mord les copeaux ou les barreaux de la cage (6).

L'intensité des stéréotypies est exprimée sous la forme d'un score compris entre 0 et 30 correspondant à la somme des valeurs obtenues sur les 5 rats d'un lot à chaque temps. La somme des scores relevés en 5 heures est calculée.

Les composés sont administrés par voie intrapéritonéale.

La dose des composés qui augmente d'environ 100 % la somme des scores en 5 heures est de 5 mg/kg pour le composé de l'exemple 1 et de 10 mg/kg pour le composé de l'exemple 2.

2) Antagonisme à l'égard des stéréotypies à l'apomorphine.

Les essais sont réalisés sur des lots de 5 rats selon un protocole inspiré de JANSSEN et col. (Arzneim. Forsch. 1965, 15, 104-117 ; 1967, 17, 841-854). Chaque animal est placé individuellement dans une boîte en plexiglas (20 × 10 × 10 cm ; NICOLET) dont le fond est recouvert d'une mince couche de frisure de bois.

Une dose de 1,5 mg/kg de chlorhydrate d'apomorphine est injectée par voie intraveineuse, 1/2 h après l'administration intrapéritonéale du composé étudié.

Les animaux sont observés pendant 1 mn, 15 mn après l'injection de l'apomorphine. Les mouvements stéréotypes de la sphère buccale sont évalués selon BOISSIER et SIMON (Thérapie, 1970, 25, 933-949) : pas de réaction caractéristique (0), quelques reniflements, léchages et mâchonnements (1), reniflements intenses et léchages continus (2), mâchonnements continus (3).

L'intensité des stéréotypies est exprimée sous la forme d'un score compris entre 0 et 15 correspondant à la somme des valeurs obtenues sur les 5 rats d'un lot, 15 minutes après l'injection de l'apomorphine.

La dose des composés qui réduit d'environ 50 % la somme des scores est de 10 à 20 mg/kg pour les composés des exemples 1à 3, de 25 mg/kg pour le composé de l'exemple 8 et de 3 mg/kg pour le composé de l'exemple 9.

3) Potentialisation de la toxicité de l'yohimbine

L'essai est réalisé selon la technique de QUINTON (Brit. J. Pharmacol. 1963, *21,* 51).

Une dose sub-léthale de 30 mg/kg de chlorhydrate d'yohimbine est injectée par voie intrapéritonéale à des lots de 10 souris mâles de 22 à 24 g.

L'injection du composé étudié a lieu par voie intrapéritonéale 1 h avant l'injection de l'yohimbine. La mortalité est relevée 24 h après l'injection de cette dernière.

A la dose de 15 mg/kg, le produit de l'exemple 2 potentialise la toxicité de l'yohimbine.

4) Antagonisme de la catalepsie induite par la prochlorpemazine

Les essais sont réalisés sur des lots de 5 rats mâles de 100 g environ.

Le composé étudié est administré par voie intrapéritonéale simultanément avec une dose de 15 mg/kg de prochlorpemazine par voie intrapéritonéale.

La catalepsie est appréciée toutes les heures pendant 7 heures suivant le test de croisement des pattes homolatérales (BOISSIER, SIMON, Therapie, 1963, *18,* 1 257-1 277) avec la cotation suivante : l'animal refuse le croisement des pattes antérieures avec les pattes postérieures homolatérales (0), il accepte le croisement recherché seulement d'un côté (0,5), il accepte le croisement des deux côtés (1).

Le composé de l'exemple 1 s'oppose à la catalepsie induite par la prochlorpemazine à une dose inférieure à 5 mg/kg ; celui de l'exemple 2 à partir de 10 mg/kg, celui de l'exemple 7 à une dose de 10 mg/kg et celui de l'exemple 5 à partir de 10 mg/kg.

5) Activité antiémétique :

L'antagonisme vis-à-vis des vomissements provoqués par l'apomorphine est étudié chez le chien (CHEN et ENSOR J. Pharmac. exp. Therap. 1959, *93,* 245-250).

Le nombre de vomissements provoqués par une injection sous cutanée de 0,1 mg/kg de chlorhydrate d'apomorphine est déterminé sur chaque animal 8 jours avant l'essai.

Le composé étudié, mis en solution aqueuse, est administré par voie sous-cutanée à des doses variables une demi-heure avant le chlorhydrate d'apomorphine.

Le composé de l'exemple 1 réduit d'environ 50 % les vomissements provoqués par l'apomorphine à la dose de 0,5 mg/kg, le composé de l'exemple 2 à la dose de 0,045 mg/kg, le composé de l'exemple 9 à la dose de 0,008 mg/kg.

6) Etude de la toxicité aiguë :

La toxicité aiguë est déterminée sur des lots de dix souris pesant 20 g environ, auquelles on administre par voie intrapéritonéale des doses croissantes du composé étudié.

La mortalité est relevée 48 heures après l'administration du composé.

La dose létale 50 ($DL_{50}$) du composé de l'exemple 1 est supérieure à 400 mg/kg ; celle du composé de l'exemple 2 à 100 mg/kg, celle des composés des exemples 4 et 9 est égale à 150 mg/kg, celle des composés des exemples 6 et 8 est égale à 200 mg/kg et celle du composé de l'exemple 7 est supérieure à 600 mg/kg.

## Revendications

1. Nouveaux dérivés du tétrahydro pyridinyl indole et leurs sels d'addition avec les acides minéraux ou organiques, caractérisés en ce qu'ils répondent à la formule générale :

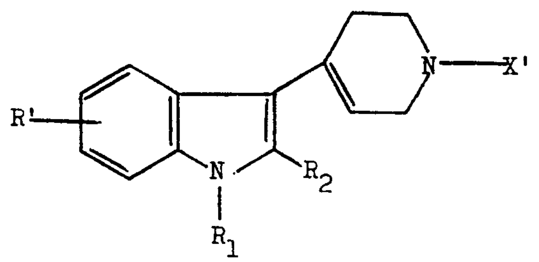

(I')

dans laquelle R' représente un atome d'hydrogène, un atome d'halogène ou un radical alcoxy renfermant de 1 à 3 atomes de carbone, $R_1$ et $R_2$, identiques ou différents, représentent un atome d'hydrogène ou un radical alcoyle renfermant de 1 à 3 atomes de carbone, X' représente un radical alcoyle renfermant de 1 à 6 atomes de carbone, un radical cycloalcoyle renfermant de 4 à 7 atomes de carbone, un radical alcényle ou alcynyle renfermant de 2 à 5 atomes de carbone, ou un radical aralcoyle renfermant de 7 à 12 atomes de carbone, étant entendu que si X' représente un radical méthyle, un au moins des substituants R', $R_1$ et $R_2$ comporte plus d'un atome de carbone, et que si X' représente un radical benzyle, un au moins des substituants R', $R_1$ et $R_2$ ne représente pas un atome d'hydrogène et R' ne représente pas un atome d'hydrogène lorsque $R_1$ et $R_2$ représentent un radical méthyle.

2. Nouveaux dérivés du tétrahydro pyridinyl indole et leurs sels d'addition avec les acides minéraux ou organiques, caractérisés en ce qu'ils répondent à la formule générale :

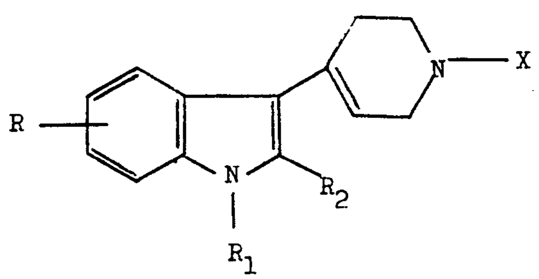

(I)

dans laquelle R représente un atome d'hydrogène ou un radical alcoxy renfermant de 1 à 3 atomes de carbone, $R_1$ et $R_2$, identiques ou différents, représentent un atome d'hydrogène ou un radical alcoyle renfermant de 1 à 3 atomes de carbone, X représente un radical alcoyle renfermant de 1 à 6 atomes de carbone, étant entendu que, si X représente un radical méthyle, un au moins des substituants R, $R_1$ et $R_2$ a plus d'un atome de carbone.

3. Dérivés du tétrahydro pyridinyl indole répondant à la formule générale (I') de la revendication 1, ainsi que leurs sels d'addition avec les acides minéraux ou organiques, caractérisés en ce que R' représente un atome d'halogène, $R_1$ et $R_2$ représentent un atome d'hydrogène ou un radical méthyle et X' représente un radical alcoyle renfermant de 2 à 6 atomes de carbone.

4. Dérivés du tétrahydro pyridinyl indole répondant à la formule générale (I') de la revendication 1, ainsi que leurs sels d'addition avec les acides minéraux ou organiques, caractérisés en ce que R' représente un atome de chlore en position 5, $R_1$ et $R_2$ représentent un atome d'hydrogène et X' représente un radical alcoyle renfermant de 2 à 6 atomes de carbone.

5. Dérivés du tétrahydro pyridinyl indole répondant à la formule générale (I) de la revendication 2, ainsi que leurs sels d'addition avec les acides minéraux ou organiques, caractérisés en ce que R représente un atome d'hydrogène ou un radical méthoxy, $R_1$ et $R_2$, identiques ou différents, représentent un atome d'hydrogène ou un radical méthyle, X représente un radical alcoyle renfermant de 2 à 6 atomes

de carbone.

6. Dérivés du tétrahydro pyridinyl indole répondant à la formule générale (I) de la revendication 2, ainsi que leurs sels d'addition avec les acides minéraux ou organiques, caractérisés en ce que R représente un atome d'hydrogène ou un radical méthoxy en position 5, $R_1$ et $R_2$ représentent un atome d'hydrogène, X représente un radical alcoyle renfermant de 2 à 6 atomes de carbone.

7. L'un quelconque des dérivés du tétrahydro pyridinyl indole répondant à la formule (I) de la revendication 2, dont les noms suivent :
— le chlorhydrate du 3-(1-propyl 1,2,3,6-tétrahydro pyridin-4-yl) 1H-indole,
— le fumarate neutre du 5-méthoxy 3-(1-propyl 1,2,3,6-tétrahydropyridin-4-yl) 1H-indole,
— le chlorhydrate du 3-(1-pentyl 1,2,3,6-tétrahydro pyridin-4-yl) 1H indole.

8. Le chlorhydrate de 5-chloro 3-(1-propyl 1,2,3,6-tétrahydro pyridin-4-yl) 1H indole.

9. L'un quelconque des dérivés du tétrahydro pyridinyl indole répondant à la formule (I') de la revendication 1, dont les noms suivent :
— le chlorhydrate de 3-(1-éthyl 1,2,3,6-tétrahydro pyridin-4-yl) 1H indole,
— le chlorhydrate de 3-/1-(1-méthyl éthyl)1,2,3,6-tétrahydro pyridin-4-yl/ 1H-indole,
— le chlorhydrate de 6-méthoxy 3-(1-propyl 1,2,3,6-tétrahydro pyridin-4-yl) 1H-indole.

10. Procédé de préparation des dérivés tels que définis par la formule générale (I') de la revendication 1, ainsi que leurs sels, caractérisé en ce que l'on fait réagir un produit de formule :

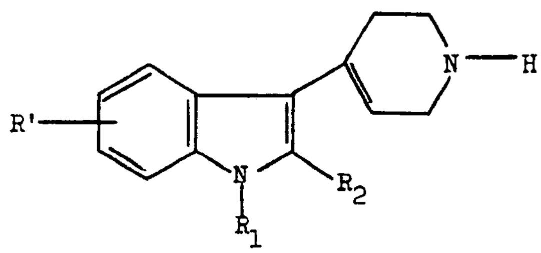

(II')

dans laquelle R', $R_1$ et $R_2$ ont la signification déjà indiquée à la revendication 1, avec un halogénure d'alcoyle de formule :

Hal—X' (III')

dans laquelle Hal représente un atome de chlore, de brome ou d'iode et X' a la signification déjà indiquée à la revendication 1, pour obtenir un produit de formule (I') que l'on isole et, le cas échéant, traite par un acide pour en former le sel.

11. Procédé selon la revendication 10, pour la préparation des dérivés tels que définis par la formule générale (I) de la revendication 2, ainsi que leurs sels, caractérisé en ce que l'on fait réagir un produit de formule :

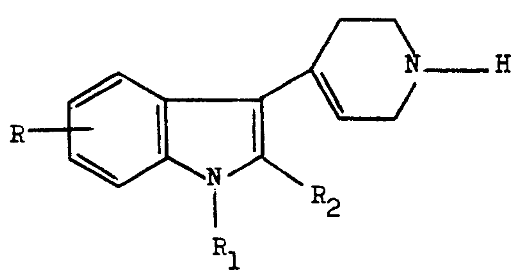

(II)

dans laquelle R, $R_1$ et $R_2$ ont la signification déjà indiquée à la revendication 2, avec un halogénure d'alcoyle de formule

Hal—X (III)

dans laquelle Hal représente un atome de chlore, de brome ou d'iode et X a la signification déjà indiquée à la revendication 2, pour obtenir un produit de formule I que l'on isole et, le cas échéant, traite par un acide pour en former le sel.

12. Médicaments, caractérisés en ce qu'ils sont constitués par les nouveaux dérivés du tétrahydro pyridinyl indole, tels que définis par la formule générale (I') de la revendication 1, ainsi que par leurs sels d'addition avec les acides pharmaceutiquement acceptables.

13. Médicaments, caractérisés en ce qu'ils sont constitués par les nouveaux dérivés du tétrahydro pyridinyl indole, tels que définis par la formule générale (I) de la revendication 2, ainsi que leurs sels

d'addition avec les acides pharmaceutiquement acceptables.

14. Médicaments, caractérisés en ce qu'ils sont constitués par les nouveaux dérivés du tétrahydro pyridinyl indole, tels que définis à l'une quelconque des revendications 3 à 6, ainsi que par leurs sels d'addition avec les acides pharmaceutiquement acceptables.

15. Médicaments, caractérisés en ce qu'ils sont constitués par les nouveaux dérivés du tétrahydro pyridinyl indole, tels que définis à la revendication 7 ou 9.

16. Médicaments, caractérisés en ce qu'ils sont constitués par le nouveau dérivé du tétrahydro pyridinyl indole tel que défini à la revendication 8.

17. Compositions pharmaceutiques, caractérisées en ce qu'elles renferment, à titre de principe actif, l'un au moins des médicaments tels que définis à l'une quelconque des revendications 12 à 16.

**Claims**

1. New derivatives of tetrahydro pyridyl indole and their addition salts with the mineral or organic acids, characterised in that they correspond to the general formula :

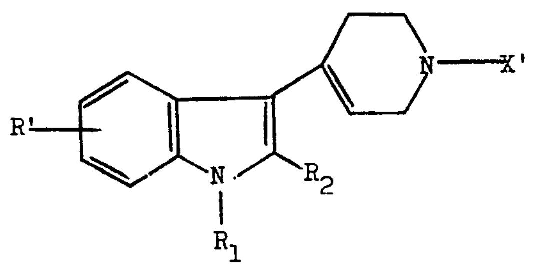

(I')

in which R' represents a hydrogen atom, a halogen atom or an alkoxy radical containing from 1 to 3 carbon atoms, $R_1$ and $R_2$, being the same or different, represent a hydrogen atom or an alkyl radical containing from 1 to 3 carbon atoms and X' represents an alkyl radical containing from 1 to 6 carbon atoms, a cycloalkyl radical containing from 4 to 7 carbon atoms, an alkenyl or alkynyl radical containing from 2 to 5 carbon atoms or an aralkyl radical containing from 7 to 12 carbon atoms, it being understood that if X' represents a methyl radical, one at least of the substituents R', $R_1$ and $R_2$ contains more than one carbon atom, and that if X' represents a benzyl radical, one at least of the substituents R', $R_1$ and $R_2$ does not represent a hydrogen atom and R' does not represent a hydrogen atom when $R_1$ and $R_2$ represent a methyl radical.

2. New derivatives of tetrahydro pyridyl indole and their addition salts with mineral or organic acids, characterised in that they correspond to the general formula :

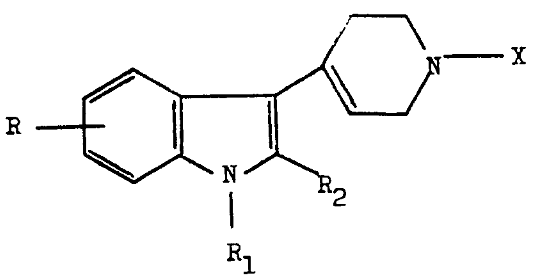

(I)

in which R represents a hydrogen atom or an alkoxy radical containing from 1 to 3 carbon atoms, $R_1$ and $R_2$, being the same or different, represent a hydrogen atom or an alkyl radical containing from 1 to 3 carbon atoms and X represents an alkyl radical containing from 1 to 6 carbon atoms, it being understood that, if X represents a methyl radical one at least of the substituents R, $R_1$ and $R_2$ has more than one carbon atom.

3. Derivatives of tetrahydro pyridyl indole corresponding to the general formula (I') of claim 1 as well as their addition salts with mineral or organic acids, characterised in that R' represents a halogen atom, $R_1$ and $R_2$ represent a hydrogen atom or a methyl radical and X' represents an alkyl radical containing from 2 to 6 carbon atoms.

4. Derivatives of tetrahydro pyridyl indole corresponding to the general formula (I') of claim 1 as well as their addition salts with mineral or organic acids, characterised in that R' represents a chlorine atom at position 5, $R_1$ and $R_2$ represents a hydrogen atom and X' represents an alkyl radical containing from 2 to 6 carbon atoms.

5. Derivatives of tetrahydro pyridyl indole corresponding to the general formula (I) of claim 2 as well as their addition salts with mineral or organic acids, characterised in that R represents a hydrogen atom or a methoxy radical, $R_1$ and $R_2$, being the same or different, represent a hydrogen atom or a methyl radical and X represents an alkyl radical containing from 2 to 6 carbon atoms.

6. Derivatives of tetrahydro pyridyl indole corresponding to the general formula (I) of claim 2 as well as their addition salts with mineral or organic acids, characterised in that R represents a hydrogen atom or a methoxy radical at position 5, $R_1$ and $R_2$ represent a hydrogen atom and X represents an alkyl radical containing from 2 to 6 carbon atoms.

7. Any one of the derivatives of tetrahydro pyridyl indole corresponding to the formula (I) of claim 2 of which the names are as follows :

— the hydrochloride of 3-(1-propyl 1,2,3,6-tetrahydro pyrid-4-yl) 1H-indole,
— the neutral fumarate of 5-methoxy 3-(1-propyl 1,2,3,6-tetrahydro pyrid-4-yl) 1H-indole, and
— the hydrochloride of 3-(1-pentyl 1,2,3,6-tetrahydro pyrid-4-yl) 1H-indole.

8. The hydrochloride of 5-chloro 3-(1-propyl 1,2,3,6-tetrahydro pyrid-4-yl) 1H-indole.

9. Any one of the derivatives of tetrahydro pyridyl indole corresponding to the formula (I') of claim 1 of which the names are as follows :

— the hydrochloride of 3-(1-ethyl 1,2,3,6-tetrahydro pyrid-4-yl) 1H-indole,
— the hydrochloride of 3-[1-(1-methyl ethyl) 1,2,3,6-tetrahydro pyrid-4-yl] 1H-indole, and
— the hydrochloride of 6-methoxy 3-(1-propyl 1,2,3,6-tetrahydro pyrid-4-yl) 1H-indole.

10. Process for preparing the derivatives as defined by the general formula (I') of claim 1 as well as their salts, characterised in that a product of formula :

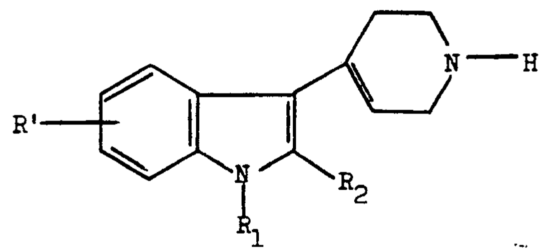

(II')

in which R', $R_1$ and $R_2$ have the meaning already indicated in claim 1, is reacted with an alkyl halide of formula :

$$Hal—X' \quad (III')$$

in which Hal represents a chlorine, bromine or iodine atom and X' has the meaning already indicated in claim 1, to obtain a product of formula (I') which is isolated and, optionally, treated with an acid to form the salt thereof.

11. Process according to claim 10 for the preparation of the derivatives as defined by the general formula (I) of claim 2 as well as their salts, characterised in that a product of formula :

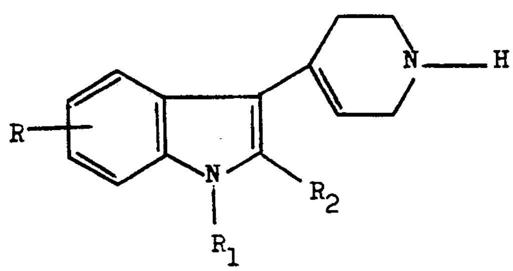

(II)

in which R, $R_1$ and $R_2$ have the meaning already indicated in claim 2, is reacted with an alkyl halide of formula

$$Hal—X \quad (III)$$

in which Hal represents a chlorine, bromine or iodine atom and X has the meaning already indicated in claim 2, to obtain a product of formula I which is isolated and, optionally, treated with an acid to form the salt thereof.

12. Medicaments, characterised in that they are constituted by the new derivatives of tetrahydro pyridyl indole, as defined by the general formula (I') of claim 1, as well as by their addition salts with

pharmaceutically-acceptable acids.

13. Medicaments, characterised in that they are constituted by the new derivatives of tetrahydro pyridyl indole, as defined by the general formula (I) of claim 2, as well as by their addition salts with pharmaceutically-acceptable acids.

14. Medicaments, characterised in that they are constituted by the new derivatives of tetrahydro pyridyl indole, as defined in any one of claims 3 to 6, as well as by their addition salts with pharmaceutically-acceptable acids.

15. Medicaments, characterised in that they are constituted by the new derivatives of tetrahydro pyridyl indole, as defined in claim 7 or 9.

16. Medicaments, characterised in that they are constituted by the new derivative of tetrahydro pyridyl indole as defined in claim 8.

17. Pharmaceutical compositions, characterised in that they contain, as active principle, one at least of the medicaments as defined in any one of claims 12 to 16.

**Ansprüche**

1. Neue Tetrahydropyridinylindolderivate und ihre Additionssalze mit Mineral- oder organischen Säuren, dadurch gekennzeichnet, daß sie der allgemeinen Formel

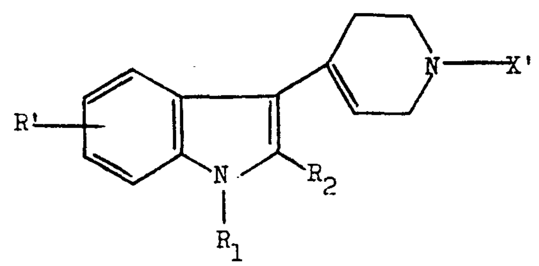

(I')

entsprechen, worin R' ein Wasserstoffatom, ein Halogenatom oder einen Alkoxyrest mit 1 bis 3 Kohlenstoffatomen bedeutet, $R_1$ und $R_2$, die gleich oder verschieden sein können, ein Wasserstoffatom oder einen Alkyrest mit 1 bis 3 Kohlenstoffatomen bedeuten, X' einen Alkyrest mit 1 bis 6 Kohlenstoffatomen, einen Cycloalkylrest mit 4 bis 7 Kohlenstoffatomen, einen Alkenyl- oder Alkinylrest mit 2 bis 5 Kohlenstoffatomen oder einen Aralkylrest mit 7 bis 12 Kohlenstoffatomen bedeutet, wobei, wenn X' einen Methylrest darstellt, zumindest einer der Substituenten R', $R_1$ und $R_2$ mehr als ein Kohlenstoffatom umfaßt und wobei, wenn X' einen Benzylrest bedeutet, zumindest einer der Substituenten R', $R_1$ und $R_2$ kein Wasserstoffatom bedeutet und R' kein Wasserstoffatom bedeutet, wenn $R_1$ und $R_2$ einen Methylrest darstellen.

2. Neue Tetrahydropyridinylindolderivate und ihre Additionssalze mit Mineral- oder organischen Säuren, dadurch gekennzeichnet, daß sie der allgemeinen Formel

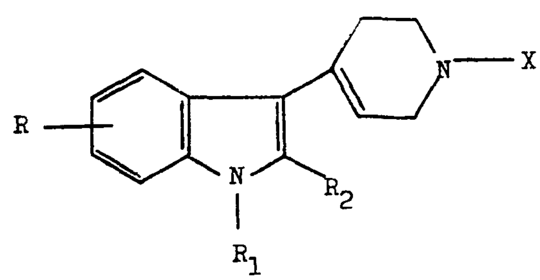

(I)

entsprechen, worin R ein Wasserstoffatom oder einen Alkoxyrest mit 1 bis 3 Kohlenstoffatomen bedeutet, $R_1$ und $R_2$, die gleich oder verschieden sein können, ein Wasserstoffatom oder einen Alkyrest mit 1 bis 3 Kohlenstoffatomen bedeuten, X einen Alkylrest mit 1 bis 6 Kohlenstoffatomen bedeutet, wobei, wenn X einen Methylrest bedeutet, zumindest einer der Substituenten R, $R_1$ und $R_2$ mehr als ein Kohlenstoffatom aufweist.

3. Tetrahydropyridinylindolderivate der allgemeinen Formel (I') gemäß Anspruch 1 sowie deren Additionssalze mit Mineral- oder organischen Säuren, dadurch gekennzeichnet, daß R' ein Halogenatom bedeutet, $R_1$ und $R_2$ ein Wasserstoffatom oder einen Methylrest bedeuten und X' einen Alkylrest mit 2 bis 6 Kohlenstoffatomen bedeutet.

4. Tetrahydropyridinylindolderivate der allgemeinen Formel (I') gemäß Anspruch 1 sowie deren

Additionssalze mit Mineral- oder organischen Säuren, dadurch gekennzeichnet, daß R' ein Chloratom in 5-Stellung darstellt, $R_1$ und $R_2$ ein Wasserstoffatom bedeuten und X' einen Alkylrest mit 2 bis 6 Kohlenstoffatomen darstellt.

5. Tetrahydropyridinylindolderivate der allgemeinen Formel (I) gemäß Anspruch 2 sowie deren Additionssalze mit Mineral- oder organischen Säuren, dadurch gekennzeichnet, daß R ein Wasserstoffatom oder einen Methoxyrest bedeutet, $R_1$ und $R_2$, die gleich oder verschieden sein können, ein Wasserstoffatom oder einen Methylrest bedeuten und X einen Alkylrest mit 2 bis 6 Kohlenstoffatomen darstellt.

6. Tetrahydropyridinylindolderivate der allgemeinen Formel (I) gemäß Anspruch 2 sowie deren Additionssalze mit Mineral- oder organischen Säuren, dadurch gekennzeichnet, daß R ein Wasserstoffatom oder einen Methoxyrest in 5-Stellung bedeutet, $R_1$ und $R_2$ ein Wasserstoffatom bedeuten und X einen Alkylrest mit 2 bis 6 Kohlenstoffatomen darstellt.

7. Eines der Tetrahydropyridinylindolderivate der Formel (I) gemäß Anspruch 2 mit den folgenden Bezeichnungen :

— 3-(1-Propyl-1,2,3,6-tetrahydropyridin-4-yl)-1H-indol-hydrochlorid,
— neutrales Fumarat des 5-Methoxy-3-(1-propyl-1,2,3,6-tetrahydropyridin-4-yl)-1H-indols,
— 3-(1-Pentyl-1,2,3,6-tetrahydropyridin-4-yl)-1H-indol-hydrochlorid.

8. 5-Chlor-3-(1-propyl-1,2,3,6-tetrahydropyridin-4-yl)-1H-indol-hydrochlorid.

9. Eines der Tetrahydropyridinylindolderivate der Formel (I') gemäß Anspruch 1 mit den folgenden Bezeichnungen :

— 3-(1-Äthyl-1,2,3,6-tetrahydropyridin-4-yl)-1H-indol-hydrochlorid,
— 3-[1-(Methyläthyl)-1,2,3,6-tetrahydropyridin-4-yl]-1H-indol-hydrochlorid,
— 6-Methoxy-3-(1-propyl-1,2,3,6-tetrahydropyridin-4-yl)-1H-indol-hydrochlorid.

10. Verfahren zur Herstellung der Derivate der allgemeinen Formel (I') gemäß Anspruch 1 sowie von deren Salzen, dadurch gekennzeichnet, daß man ein Produkt der Formel

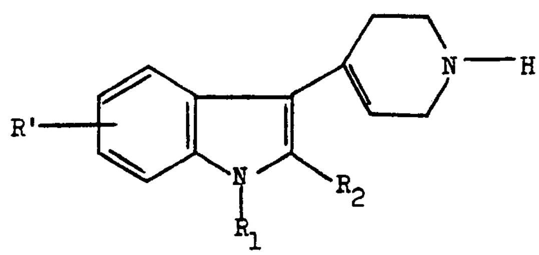

(II')

worin R', $R_1$ und $R_2$ die in Anspruch 1 angegebene Bedeutung besitzen, mit einem Alkylhalogenid der Formel

Hal—X' (III')

worin Hal ein Chlor-, Brom- oder Jodatom bedeutet und X' die in Anspruch 1 angegebene Bedeutung besitzt, umsetzt, um ein Produkt der Formel (I') zu erhalten, welches man isoliert und gegebenenfalls mit einer Säure behandelt, um hieraus das Salz zu bilden.

11. Verfahren gemäß Anspruch 10 zur Herstellung der Derivate der allgemeinen Formel (I) gemäß Anspruch 2 sowie von deren Salzen, dadurch gekennzeichnet, daß man ein Produkt der Formel

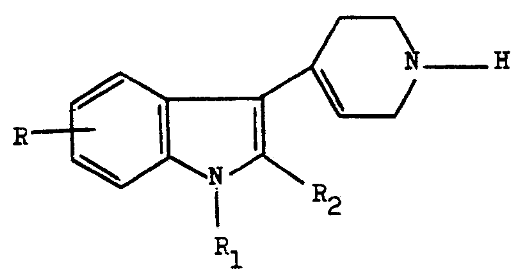

(II)

worin R, $R_1$ und $R_2$ die in Anspruch 2 angegebene Bedeutung besitzen, mit einem Alkylhalogenid der Formel

Hal—X (III)

worin Hal ein Chlor-, Brom- oder Jodatom bedeutet und X die in Anspruch 2 angegebene Bedeutung

besitzt, umsetzt, um ein Produkt der Formel (I) zu erhalten, welches man isoliert und gegebenenfalls mit einer Säure behandelt, um hieraus das Salz zu bilden.

12. Arzneimittel, dadurch gekennzeichnet, daß sie aus den neuen Tetrahydropyridinylindolderivaten der allgemeinen Formel (I') gemäß Anspruch 1 sowie aus deren Additionssalzen mit pharmazeutisch verträglichen Säuren bestehen.

13. Arzneimittel, dadurch gekennzeichnet, daß sie aus den neuen Tetrahydropyridinylindolderivaten der allgemeinen Formel (I) gemäß Anspruch 2 sowie aus deren Additionssalzen mit pharmazeutisch verträglichen Säuren bestehen.

14. Arzneimittel, dadurch gekennzeichnet, daß sie aus den neuen Tetrahydropyridinylindolderivaten gemäß einem der Ansprüche 3 bis 6 sowie aus deren Additionssalzen mit pharmazeutisch verträglichen Säuren bestehen.

15. Arzneimittel, dadurch gekennzeichnet, daß sie aus den neuen Tetrahydropyridinylindolderivaten gemäß Anspruch 7 oder 9 bestehen.

16. Arzneimittel, dadurch gekennzeichnet, daß sie aus dem neuen Tetrahydropyridinylindolderivat gemäß Anspruch 8 bestehen.

17. Pharmazeutische Zusammensetzungen, dadurch gekennzeichnet, daß sie als Wirkstoff zumindest eines der Arzneimittel gemäß einem der Ansprüche 12 bis 16 enthalten.